# EUROPEAN PATENT APPLICATION

(11) **EP 2 022 796 A1**
(43) Date of publication of application: **11.02.2009**
(21) Application number: 07113961.2
(22) Date of filing: 07.08.2007
(51) Int. Cl.: C07J 71/00, A61K 31/58, A61P 11/06, A61P 5/44

(54) **Amorphous ciclesonide**

(71) Applicant: Nycomed GmbH, 78467 Konstanz (DE)
(72) Inventor: Feth, Martin Dr., 65779 Kelkheim-Hornau (DE); Hummel, Rolf-Peter, 78315 Radolfzell (DE); Volz, Jürgen, 78315 Radolfzell (DE); Zimmermann, Peter, 78467 Konstanz (DE); Schmidt, Beate, 78476 Allensbach (DE)
(74) Representative: Mechnich, Oliver M.J.

(57) **Abstract**

The present invention relates to amorphous ciclesonide, methods to prepare the same, pharmaceutical compositions comprising amorphous ciclesonide as active ingredient, and methods of treatment using amorphous ciclesonide.

## Description

### Technical Field

The present invention relates to amorphous ciclesonide and, more particularly, to amorphous ciclesonide which is suitable for use in the preparation of pharmaceutical compositions. Further subject matter of the invention are processes of preparing the same. The invention also relates to pharmaceutical compositions containing amorphous ciclesonide as active ingredient, and to methods of treatment using the same.

### Background Art

16,17-[(Cyclohexyl-methylene)bis(oxy)]-11-hydroxy-21-(2-methyl-1-oxopropoxy)-pregna-1,4-diene-3,20-dione[11β,16α], also known by the name ciclesonide, is a compound represented by formula I:

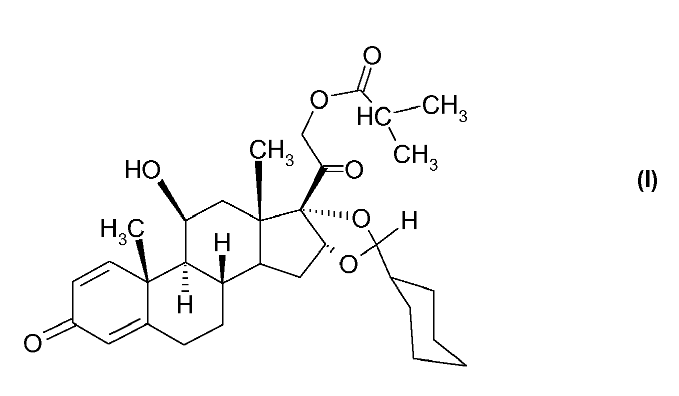

The compound is known from, *inter alia,* US patent 5,482,934. As an inhalable corticosteroid, ciclesonide is useful in the treatment of inflammatory conditions. In particular, ciclesonide is useful in the treatment of respiratory diseases, particularly as an anti-asthmatic agent and as an agent for treatment of allergic rhinitis.

Formulated as an aerosol for treatment of asthma, ciclesonide is marketed under the name Alvesco. An alternative formulation as nasal spray for treatment of seasonal and perennial allergic rhinitis is planned to be marketed in the United States under the name Omnaris/Omnair.

Methods for epimer enrichment (WO 98/09982), the preparation of crystalline ciclesonide with specific particle size distributions (WO 2004/085460), the preparation of aqueous suspensions of ciclesonide for nebulisation (WO 2005/058935), and the preparation of compositions comprising ciclesonide, propellant (such as 1,1,1,2-tetrafluoroethane, 1,1,1,2,3,3,3-heptafluoropropane, or a mixture thereof) and ethanol (WO 98/52542) have been reported.

### Disclosure of the Invention: Technical problem

In line with the highly hydrophobic character of the ciclesonide molecule (see formula I), this compound has a relatively poor solubility in water. The crystalline form of ciclesonide is characterized by a solubility of about 50 µg/l after 1 hour. Solubility after 24 hours is about 64 µg/l, and the saturation solubility is about 91 µg/l. While it has to be noted that solubility after a given time shorter than the time for reaching equilibrium will depend on the particular conditions of the dissolution experiment, it is clear that bioavailability of the active agent of pharmaceutical preparations containing crystalline ciclesonide is limited by the poor solubility and the slow dissolution kinetics of the crystalline form. Accordingly, the problem to be solved was to provide an alternative form of ciclesonide characterized by a better solubility in water. A further problem to be solved was to provide methods of synthesizing such forms characterized by a better dissolution behaviour.

### Disclosure of the Invention: Technical solution

In a first aspect, the invention relates to an amorphous form of ciclesonide or a pharmaceutically acceptable solvate thereof. Amorphous ciclesonide can be characterized by its powder X-ray diffraction pattern (Fig. 1), FT-Raman spectrum (Figs. 2 and 3) or DSC curve (Fig. 4).

Accordingly, the invention relates to ciclesonide or a pharmaceutically acceptable solvate thereof, being to more than 10% (w/w) in amorphous form. In particular, the invention relates to ciclesonide or a pharmaceutically acceptable solvate thereof, being to more than 20% (w/w), to more than 30% (w/w) or to more than 40% (w/w) in amorphous form. Preferably, the invention relates to ciclesonide or a pharmaceutically acceptable solvate thereof, being to more than 50% (w/w) in amorphous form. Even more preferably, the invention relates to ciclesonide or a pharmaceutically acceptable solvate thereof, being to more than 60% (w/w) in amorphous form. Even more preferably, the invention relates to ciclesonide or a pharmaceutically acceptable solvate thereof, being to more than 70% (w/w) in amorphous form. Even more preferably, the invention relates to ciclesonide or a pharmaceutically acceptable solvate thereof, being to more than 80% (w/w) in amorphous form. Even more preferably, the invention relates to ciclesonide or a pharmaceutically acceptable solvate thereof, being to more than 90% (w/w) in amorphous form. In a most preferred embodiment, the invention relates to ciclesonide or a pharmaceutically acceptable solvate thereof, being to more than 95% (w/w) in amorphous form. In a second most preferred embodiment, the invention relates to ciclesonide or a pharmaceutically acceptable solvate thereof, being to more than 99% (w/w) in amorphous form.

In a second aspect, the invention relates to a process for preparing amorphous ciclesonide, comprising providing a solution of the substance of formula I or a pharmaceutically acceptable solvate thereof and removing the solvent by lyophilization or spray drying.

In a third aspect, the invention relates to a process for preparing amorphous ciclesonide, comprising the step of milling crystalline ciclesonide.

According to the invention, the name "ciclesonide" is understood as meaning the pure R epimer of the compound of formula I ([11β,16α]16,17-[(cyclohexylmethylene)bis(oxy)]-11-hydroxy-21-(2-methyl-1-oxopropoxy)pregna-1,4-diene-3,20-dione) as well as R/S epimer mixtures in any desired mixing ratio (that is the compounds [11β,16α(R)]-16,17-(cyclohexylmethylene)bis(oxy)]-11-hydroxy-21-(2-methyl-1-oxo-propoxy)-pregna-1,4-diene-3,20-dione and [11β,16α(S)]-16,17-[(cyclohexylmethylene)bis(oxy)]-11-hydroxy-21-(2-methyl1-oxopropoxy)pregna-1,4-diene-3,20-dione), those being preferred which essentially consist of R epimers. According to the invention, essentially consisting of R epimers means that the proportion of S epimers in the mixture is less than or equal to 5% by weight, preferably less than or equal to 1% by weight.

### Disclosure of the Invention: Advantageous effects

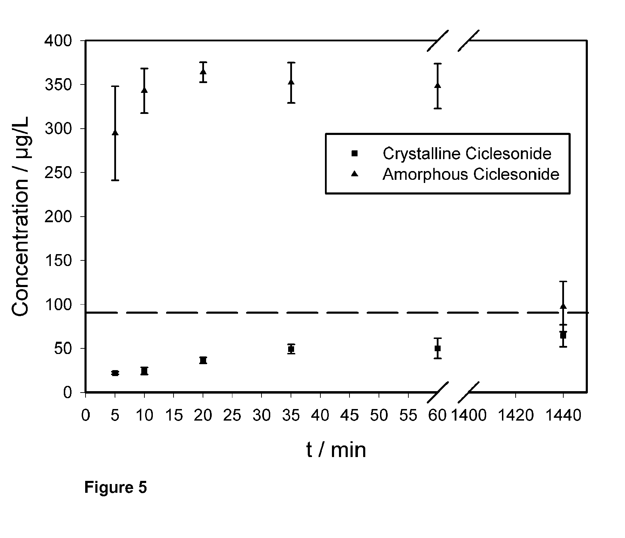

It was surprisingly found that amorphous ciclesonide exhibts dissolution kinetics that is completely different from the dissolution kinetics of crystalline ciclesonide. When dissolving crystalline ciclesonide known from the art in water, the concentration of dissolved ingredient increases very slowly and approaches the plateau defined by the saturation solubility (91 µg/l ± 10 µg/l). Concentration of dissolved ciclesonide is 64 µg/l ± 10 µg/l after 24 hours. In complete contrast, the concentration of dissolved ingredient upon dissolution of amorphous ciclesonide soars up to 290 µg/l ± 50 µg/l within 5 minutes, followed by an even further increase of the concentration to 340 µg/l ± 20 µg/l after 20 minutes (Fig. 5). Subsequently, the concentration begins to slowly decrease again, but still is about sevenfold higher than the concentration of dissolved ingredient when crystalline ciclesonide is dissolved after 1 hour. Later on, the concentration decreases further and reaches 105 µg/l ± 20 µg/l after 24 hours which is close to the saturation solubility. Thus, the bioavailability of amorphous ciclesonide according to the invention should initially be severalfold higher than the bioavailability of crystalline ciclesonide known from the art, and this effect will be maintained for a considerable time span. It can be expected that such an enhanced bioavailability of amorphous ciclesonide is highly beneficial in terms of the onset of the desired clinical effect, even more given the fact that this onset is subject to several hours of delay with pharmaceutical preparations containing crystalline ciclesonide characterized by slow dissolution and a very low initial solubility. On the other hand, the advantage of an increased solubility and its effect on bioavailability of the compound does not limit the ease in processing the amorphous material, including, for example, ease of equipment cleaning, ease in formulation and delivery of the material.

A further advantage of amorphous ciclesonide according to the invention is its low bulk density which can be useful when, as part of a formulation process, ciclesonide is to be uniformly and homogenously coated around other particles such as, e.g., starch grains.

It was also found that amorphous ciclesonide according to the invention is stable under pressure and does not tend to change its structure when subjected to elevated pressure. Amorphous ciclesonide was subjected to a pressure of up to 2.500 kg/cm³ for 5 min and did not show any signs of recrystallization. Such a stability against pressure is particularly desirable when a pharmaceutical agent is used for preparation of solid pharmaceutical compositions such as tablets or granules.

Furtheron, despite the low bulk density, it was surprisingly found that amorphous ciclesonide according to the invention is not hygroscopic.

### Brief Description of Drawings

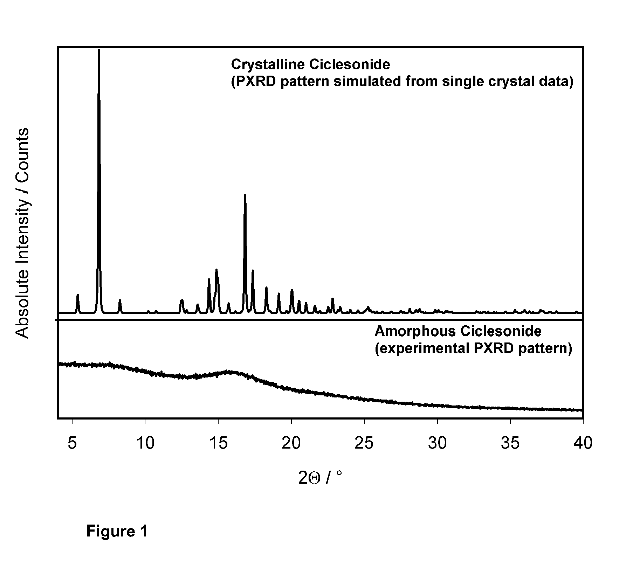

- Figure 1:: Powder X-ray diffraction pattern of amorphous ciclesonide as prepared in example 5 in comparison with the PXRD pattern of crystalline Ciclesonide, obtained from simulation of single crystal data, in the 2Θ range between 3 and 40°.

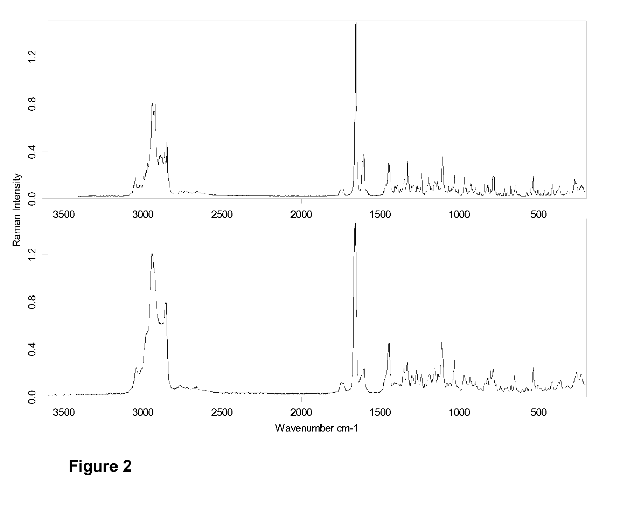

- Figure 2:: Comparison of the FT-Raman spectra of amorphous (spectrum on bottom; substance prepared as in example 5) and crystalline (spectrum on top) Ciclesonide in the wavenumber range between 200 - 3600 cm⁻¹.

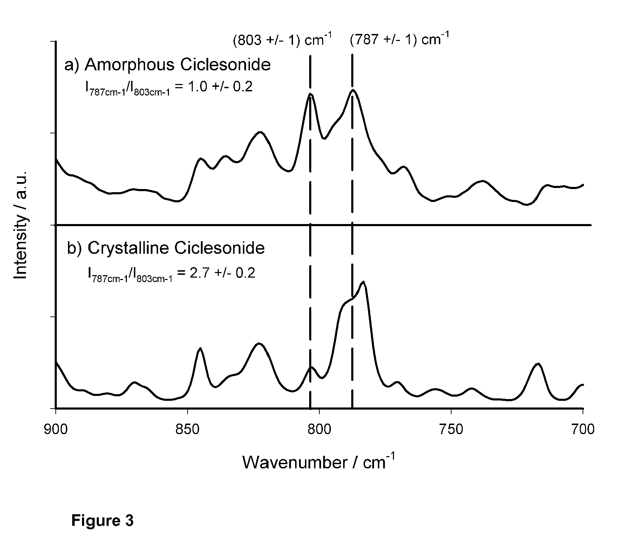

- Figure 3:: Comparison of the FT-Raman spectra of amorphous and crystalline Ciclesonide as prepared in example 5 in the wavenumber range between 700 - 900 cm⁻¹.

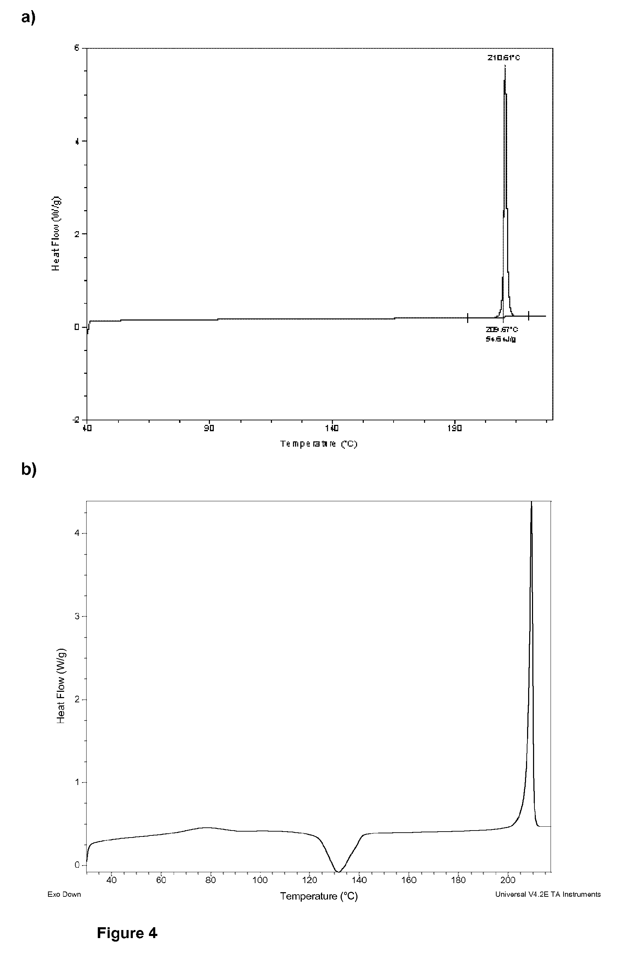

- Figure 4:: DSC curve of a) crystalline and b) amorphous ciclesonide as prepared in example 5. The heating rate was 10 K/min.
- Figure 5:: Dissolution behaviour of crystalline (■) and amorphous (▲) ciclesonide as prepared in example 5 in water at 37°C, monitored over 60 minutes and after 24 hours. The saturation solubilities measured with amorphous (91.6 ± 5.2 µg/l) and crystalline (90.1 ± 2.2 µg/l) ciclesonide in water at 37°C are marked as dashed line in the graph.

### Modes for Carrying Out the Invention

The present invention provides a novel amorphous form of ciclesonide and processes for the preparation thereof.

As used herein, the term "amorphous" refers to a solid body devoid of long-range crystalline order. Such a lack of crystalline order can be detected and monitored, e.g., by X-ray diffraction (XRD), FT-Raman spectroscopy, and differential scanning calorimetry (DSC). Accordingly, amorphous ciclesonide can be characterized by its X-ray diffraction pattern (Fig. 1), FT-Raman spectrum (Figs. 2 and 3), and DSC curve (Fig. 4).

In a first aspect, the invention relates to amorphous ciclesonide, being to more than 50% by weight in amorphous form. In particular, the invention relates to an amorphous form of ciclesonide, obtainable by at least one of the processes described hereinbelow. The invention also relates to an amorphous form of ciclesonide, obtained by one of the processes described hereinbelow.

Accordingly, the invention relates to an amorphous form of a pharmaceutically acceptable solvate of ciclesonide, obtainable by at least one of the processes described hereinbelow. The invention also relates to an amorphous form of a pharmaceutically acceptable solvate of ciclesonide, obtained by one of the processes described hereinbelow.

Accordingly, the invention relates to ciclesonide, characterized by an X-ray diffraction pattern essentially devoid of sharp Bragg reflexes as shown in Fig. 1. In particular, the invention relates to ciclesonide, characterized by an X-ray diffraction pattern devoid of the six prominent Bragg reflexes in the powder X-ray diffraction pattern of crystalline ciclesonide, collected using the Kα₁ line with a wavelength of 1.54056 Å of a copper X-ray tube as radiation source, at 2Θ = 6.8° +/-0.3°, 14.4° +/-0.3°, 14.9° +/-0.3°, 16.8° +/-0.3°, 17.4° +/-0.3°, and 18.3° +/-0.3°. The invention also relates to pharmaceutically acceptable solvates of ciclesonide, characterized by an X-ray diffraction pattern essentially devoid of sharp Bragg reflexes as shown in Fig. 1.

Accordingly, the invention relates to ciclesonide, characterized by a FT-Raman spectrum essentially as shown in Figs. 2 and 3. Amorphous ciclesonide has a Raman signal ratio of I _{787 cm-1} /I _{803 cm-1} = 1.0 +/-0.2, whereas crystalline ciclesonide is characterized by a ratio of I _{787 cm-1} /I _{803 cm-1} = 2.7 +/-0.2 (see Fig. 3). I _{787 cm-1} means the relative signal intensity at the wavenumber 787 cm⁻¹, I _{803 cm-1} means the relative signal intensity at the wavenumber 803 cm⁻¹. Thus, the invention also relates to ciclesonide characterized by a FT-Raman spectrum having a signal ratio of I _{787 cm-1} /I _{803 cm-1} = 1.0 +/-0.2.

Accordingly, the invention relates to ciclesonide, characterized by a DSC scan essentially as shown in Fig. 4. Amorphous or partially amorphous ciclesonide show characteristic exothermal recrystallisation signals below the melting point of crystalline ciclesonide (209 - 212°C). Thus, the invention also relates to ciclesonide characterized by a DSC scan having an exothermal recrystallization signal below the melting point of crystalline ciclesonide. In particular, the invention relates to ciclesonide characterized by a DSC scan devoid of extothermal recrystallization signals in the range of 80 to 180°C, preferrably in the range of 100 to 150°C.

The invention also relates to an amorphous form of ciclesonide as described above, characterized by a solubility in water at 37°C after 20 min of at least 150 µg/l.

The invention also relates to an amorphous form of ciclesonide as described above, characterized by a solubility in water at 37°C after 20 min of at least 300 µg/l.

The invention also relates to an amorphous form of ciclesonide as described above, characterized by a solubility in water at 37°C after 1 h of at least 150 µg/l.

The invention also relates to an amorphous form of ciclesonide as described above, characterized by a solubility in water at 37°C after 1 h of at least 300 µg/l.

The solubility values given above refer to solubilities determined under the experimental conditions mentioned in example 3.

In a second aspect, the invention provides a process for the preparation of amorphous ciclesonide.

In a first embodiment, the process includes the preparation of a solution of ciclesonide in a solvent or a mixture of solvents. The pure amorphous ciclesonide according to the invention can be obtained by the removal of the solvent or the mixture of solvents used by lyophilizing or spray drying.

The solution of ciclesonide can be prepared by dissolving crystalline ciclesonide in a solvent or a mixture of solvents. A further possibility is the direct use of the solution of the last reaction step of the synthesis in which ciclesonide is formed.

The preparation of a ciclesonide solution may include the usage of an ultrasonic bath or the heating of the solvent during the dissolution process.

The crystalline ciclesonide used as starting material includes the pure forms (including possible polymorphs) as well as solvates, hydrates, and mixtures thereof. Ciclesonide may be obtained by methods in the art, such as the processes reported in US-A 5,482,934.

Possible solvents which may be used for the process include one or more of alcohols, ketones, ethers, chlorinated hydrocarbons, esters, cyclic ethers, and mixtures thereof. Alcohols to be used in the process according to the invention may include methanol, ethanol, isopropanol, 2-methyl-propan-2ol (tert. butanol), and mixtures thereof. Ketones to be used in the process according to the invention may include acetone, ethyl methyl ketone, methyl isobutyl ketone, diisobutyl ketone, and mixtures thereof. Ethers to be used in the process according to the invention may include diethylether, diisopropylether, tertiary butylethylether, and mixtures thereof. Chlorinated hydrocarbons to be used in the process according to the invention may include one or more of dichloromethane, dichloroethane, and mixtures thereof. Esters to be used in the process according to the invention may include one or more of methyl acetate, ethyl acetate, isopropyl acetate, n-propyl acetate, isobutyl acetate, butyl acetate, amyl acetate, and mixtures thereof. Cyclic ethers to be used in the process according to the invention may include tetrahydrofurane, dioxane, and mixtures thereof.

Accordingly, the invention relates to a process for preparing amorphous ciclesonide, comprising the steps of:
a. providing a solution of the compound of formula I or a pharmaceutically acceptable solvate thereof,
b. quench cooling the solution of step (a), and
c. lyophilizing the quench cooled solution.

Quench cooling means to subject the solution of ciclesonide as obtained in step (a) to rapid cooling, the cooling rate being at least >100 °C/min, down to a temperature of -20°C or less.

Accordingly, the invention relates to a process for preparing amorphous ciclesonide, comprising the steps of:
a. providing a solution of the compound of formula I or a pharmaceutically acceptable solvate thereof, and
b. spray drying the solution of step (a).

Preferably, the invention relates to a process for preparing amorphous ciclesonide as outlined above, comprising the use of a solvent for preparing the solution of the substance of formula I or a pharmaceutically acceptable solvate thereof, the solvent being selected from tert-butanol and dioxan.

In a second embodiment, amorphous ciclesonide is prepared by milling or micronization until the crystalline ciclesonide is converted to amorphous material, as can be determined by XRD, FT-Raman spectroscopy or DSC. The milling process can be a dry milling or a wet milling process. However, dry milling is preferred. The milling procedure may be carried out by milling machines known in the art. Suitable milling machines include various types of ball mills, roller mills, cryo mills, gyratory mills, and the like.

Accordingly, the invention relates to a process for preparing an amorphous pharmaceutically acceptable solvate of ciclesonide, comprising the step of milling a crystalline pharmaceutically acceptable solvate of ciclesonide.

In a third aspect, the invention relates to the use of amorphous ciclesonide according to any of the above specifications for the preparation of a pharmaceutical composition. In a preferred embodiment, the pharmaceutical composition is an inhalable dry powder, an inhalable liquid or a liquid that can be administered as a spray.

In a fourth aspect, the invention relates to a pharmaceutical composition, comprising amorphous ciclesonide according to any of the above specifications as active ingredient.

In a fifth aspect, the invention relates to amorphous ciclesonide according to any of the above specifications for therapeutic use. In a preferred embodiment, the therapeutic use is selected from an inflammatory condition, a respiratory disease, asthma, and allergic rhinitis.

In a sixth aspect, the invention relates to a method of treatment comprising administering to a patient in need thereof a pharmaceutical composition comprising amorphous ciclesonide according to the above specifications.

### Industrial applicability

Amorphous ciclesonide according to the invention and pharmaceutical compositions comprising the same are particularly suitable in the prophylaxis and/or treatment of respiratory diseases and, in general, in the prophylaxis and treatment of clinical conditions for which a glucocorticosteroid is indicated. Respiratory disease according to the invention includes in particular diseases associated with inflammatory airway conditions and/or reversible airways obstruction such as asthma, nocturnal asthma, exercise-induced asthma, chronic obstructive pulmonary diseases (COPD) (e. g. chronic and wheezy bronchitis, emphysema), croup, respiratory tract infection and upper respiratory tract disease (e. g. rhinitis, such as allergic and seasonal rhinitis).

The compositions comprising amorphous ciclesonide (also referred to as formulations) include those suitable for oral, parenteral including subcutaneous, intradermal, intramuscular, intravenous and intraarticular, intranasal, inhalation (including fine particle dusts or mists which may be generated by means of various types of metered dose pressurised aerosols, nebulisers or insufflators), rectal and topical (including dermal, buccal, sublingual and intraocular administration), although the most suitable route may depend upon for example the condition and disorder of the recipient. The formulations may conveniently be presented in unit dosage form and may be prepared by any of the methods well known in the art of pharmacy. All methods include the step of bringing the active ingredients into contact with the carrier, which constitutes one or more accessory ingredients/excipients.

Exemplary doses in connection with the invention comprise 20, 40, 60, 80, 100, 120, 140, 160, 180 or 200 µg amorphous ciclesonide. Preferably the dose comprises 40, 80 or 160 µg amorphous ciclesonide. The dose is preferably a daily dose and administered once or twice daily, preferably once daily. A once daily dose may be administered any time of the day, e.g. in the morning or preferably in the evening. The administration of a daily dose of amorphous ciclesonide in the range of from 20 to 200 µg is preferably part of a continuous treatment regimen, preferably a treatment period of more than one day, particularly preferably more than one week, e.g. a two week treatment period, a one month treatment period, a one year treatment period or a life long treatment period.

In one embodiment amorphous ciclesonide is provided in a form suitable for inhalation. Formulations for inhalation include powder compositions, which will preferably contain lactose, and spray compositions which may be formulated, for example, as suspensions or as aerosols delivered from pressurized packs, with the use of a suitable propellant, e.g. 1,1,1,2-tetrafluorethane, 1,1,1,2,3,3,3-heptafluoropropane, carbon dioxide or another suitable gas. A class of propellants which are believed to have minimal ozone-depleting effects in comparison to conventional chlorofluorocarbons comprise hydrofluorocarbons and a number of medicinal aerosol formulations using such propellant systems are disclosed in, for example, EP 0 372 777, WO 91/04011, WO 91/11173, WO 91/11495, WO 91/14422, WO 93/11743, and EP 0 553 298. These applications are all concerned with the preparation of pressurised aerosols for the administration of medicaments and seek to overcome problems associated with the use of this new class of propellants, in particular the problems of stability associated with the pharmaceutical formulations prepared. The applications propose, for example, the addition of one or more of excipients such as polar cosolvents or wetting agents (e.g. alcohols such as ethanol), alkanes, dimethyl ether, surfactants (including fluorinated and non-fluorinated surfactants, carboxylic acids such as oleic acid, polyethoxylates etc.) or bulking agents such as a sugar (see for example WO 02/30394) and vehicles such as cromoglicic acid and/or nedocromil which are contained at concentrations, which are not therapeutically and prophylactically active (see WO 00/07567). For suspension aerosols, the active ingredients should be micronized so as to permit inhalation of substantially all of the active ingredients into the lungs upon administration of the aerosol formulation, thus the active ingredients will have a mean particle size of less than 100 microns, desirably less than 20 microns, and preferably in the range 0.7 to 10 microns, for example, 1 to 5 microns.

Ciclesonide is preferably present in the formulation at a concentration which allows administration of a dose of from 20 to 200 µg. The propellant preferably includes a hydrofluoroalkane, in particular Propellant 134a, Propellant 227 or a mixture thereof. In the case of a mixture the ratio of Propellant 134a to Propellant 227 is generally in a range from 75:25 w/w to 25:75 w/w. The formulations may contain surfactant such as oleic acid, but may be also free of surfactant. The formulations are preferably free of other excipients.

In a preferred embodiment of the invention the pharmaceutical formulation comprises the amorphous ciclesonide as a dry powder, i.e. ciclesonide is present in a dry powder comprising finely divided amorphous ciclesonide optionally together with a finely divided pharmaceutically acceptable carrier, which is preferably present and may be one or more materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose, particularly in the form of the monohydrate. The dry powder may be in capsules made of, e.g., gelatine or plastic, or in blisters, for use in a dry powder inhalation device, preferably in dosage units of the ciclesonide together with the carrier in amounts to bring the total weight of powder in each capsule to from 5mg to 50mg. Alternatively the dry powder may be contained in a reservoir of a multi-dose dry powder inhalation device. Capsules and cartridges of for example gelatin, or blisters of for example laminated aluminium foil, for use in an inhaler or insulator may be formulated containing a powder mix of the active ingredients and a suitable powder base such as lactose or starch, preferably lactose. In this aspect, the active ingredient is suitably micronized so as to permit inhalation of substantially all of the active ingredients into the lungs upon administration of the dry powder formulation, thus the active ingredient will have a particle size of less than 100µm, desirably less than 20µm, and preferably in the range 1 to 10µm. The solid carrier, where present, generally has a maximum particle diameter of 300µm, preferably 200µm, and conveniently has a mean particle diameter of 40 to 100µm, preferably 50 to 75µm. If required, the particle size of the active ingredient and/or that of a solid carrier where present in dry powder compositions can be reduced to the desired level by conventional methods, for example by grinding in an air-jet mill, ball mill or vibrator mill.

Where the inhalable form of the composition of the invention is the finely divided particulate form, the inhalation device may be, e.g., a dry powder inhalation device adapted to deliver dry powder from a capsule or blister containing a dosage unit of the dry powder or a multi-dose dry powder inhalation device. Such dry powder inhalation devices are known in the art. Examples which may be mentioned are Cyclohaler®, Diskhaler® Rotadisk®, Turbohaler® or the dry powder inhalation devices disclosed EP 0 505 321, EP 0 407 028, EP 0 650 410, EP 0 691 865 or EP 0 725 725 (Ultrahaler®).

Formulations for inhalation by nebulization may be formulated with an aqueous vehicle with the addition of agents such as acid or alkali, buffer salts, isotonicity adjusting agents or antimicrobials. They may be sterilized by filtration or heating in an autoclave. Suitable technologies for this type of administration are known in the art. As an example the Mystic® technology is to be mentioned (see for example US 6,397,838, US 6,454,193 and US 6,302,331).

Preferred unit dosage formulations are those containing a pharmaceutical effective dose, as hereinbefore recited, or an appropriate fraction thereof, of the active ingredient. Thus, in the case of formulations designed for delivery by metered dose pressurised aerosols, one actuation of the aerosol may deliver half of the therapeutical effective amount such that two actuations are necessary to deliver the therapeutically effective dose.

It should be understood that in addition to the ingredients particularly mentioned above, the formulations of this invention may include other agents conventional in the art having regard to the type of formulation in question. Furthermore, the claimed formulations include bioequivalents as defined by the US Food and Drugs Agency.

The following examples are intended to illustrate the invention and should not be construed as limiting the scope of the invention in any way.

### Examples

### Example 1: PXRD, DSC, TG and ¹H-NMR Spectroscopy

The X-ray diffraction (XRD) experiments in reflection mode were performed on a STOE STADI P diffractometer equipped with a Ge(111) monochromator and a linear PSD detector over an angular range of 2θ = 3-40° (angular step size: 0.2°, measurement time per step: 15 s). For the measurements Cu K_{α1} radiation (λ_{Kα1} = 1.54056 A, E_{Kα1} = 8047.78 eV, U = 40 kV, I = 30 mA) of an X-ray tube was used. The ciclesonide powders were filled between sheets of mylar foil and adjusted in the path of rays for the transmission mode experiments. Figure 1 shows XRD pattern obtained from amorphous and crystalline ciclesonide, respectively.

The DSC scans of the investigated batches were carried out in a temperature range between 40 - 220°C on a dynamical differential scanning calorimeter of TA instruments (DSC Q1000) with dry nitrogen as purge gas (50 mL/min). Heating rates of 10 K/min and 100 K/min were used for the determination of the calibration curves (amorphous content vs. recrystallisation enthalpy), heating rate of 10, 20, 30, 50 and 100 K/min were used for the screening of the detection limit (1 % [w/w] amorphous content). Before performing the DSC sample scans, a T4P calibration (baseline, cell constant, onset slope, cp constant, indium melting point temperature calibration) was carried out for the heating rates 10 K/min and 100 K/min. Figure 4 shows DSC scans obtained from amorphous and crystalline ciclesonide, respectively.

To determine the residual amount of tert-Butanol of amorphous ciclesonide as obtained in example 5, thermogravimetric measurements of amorphous ciclesonide were performed on a Hi-Res TGA 2950 thermogravimetric Analyzer from TA instruments. For each measurement about 5 mg of the substance were accurately weighed into a platinum TG pan. The weighed change of the sample was recorded in a temperature range between 30°C and 250°C applying a heating rate of 10 K/min. ¹H-NMR spectra of amorphous ciclesonide were recorded in CDCl₃ on a Bruker 200 MHz-NMR spectrometer (DPX 200). For the results, see example 5.

### Example 2: FT-Raman Spectroscopy

FT-Raman spectra were recorded on a Bruker Optics RAM II module, which was coupled to a Bruker Optics Vertex 70 FT-IR spectrometer. The RAM II module was equipped with a liquid nitrogen cooled, high sensitivity germanium detector (D-418 TF) and a diode pumped 500 mW Nd:YAG laser (wavelength: 1064 nm). Data acquisition and analysis was performed using the software package OPUS 5.4 from Bruker Optics. For each spectrum 512 scans were collected in 180° reflection mode using a spectral resolution of 2 cm⁻¹ in order to provide Raman spectra with a low S/N-ratio in combination with well-resolved Raman signals. To avoid heating of the colourless samples, laser power was set to a value of 100 mW. The sample preparation consisted of gently compacting the powders into a cylindrical aluminium sample pan (height: 4 mm, diameter: 10 mm) with a pinhole (diameter: 2 mm, depth: 1 mm). Figures 2 and 3 show Raman spectra obtained from amorphous and crystalline ciclesonide, respectively.

### Example 3: Dissolution Experiments

For each timepoint (5 min, 10 min, 20 min, 35 min, 60 min and 1440 min) of the dissolution experiment, three samples each of X-ray amorphous and crystalline ciclesonide were prepared by weighing a defined amount of in each case about 15 mg into a 250 ml conical wide-necked Erlenmeyer flask and stirring (850 rpm, glass coated magnetic stir bar, having a length of 2.4 cm and a diameter of 0.59 cm) the samples at 37°C in 100 ml water (Millipore quality, ELIX 3, Gradient A 10, pH 5.92 - 6.00). The moment of the addition of water was defined as to. The tempering of the samples was performed using an air bath. After the desired stirring time, the complete samples were filtered through a 0.2 µm membrane filter (Schleicher & Schuell, RC 0.2 µm Spartan 30/A) and concentrated on a Sep-Pak cartridge (Waters Sep-Pak cartridge C18, WAT051910). The cartridge was then eluted with ethanol (gradient grade, Merck). The ciclesonide in the ethanol solution was then quantified by HPLC (LaChrom®-HPLC System Series 7000, column: Zorbax SB phenyl, eluent: water/ethanol 38 % : 62 % (v/v) isocratic, column temperature: 60°C, injection volume: 50 µL, UV-detection at 243 nm) against an external standard. Results are given in Figure 4.

### Example 4: Saturation Solubility Experiments

Defined amounts of in each case about 14 to 25 mg of X-ray amorphous or crystalline ciclesonide were weighed into a conical Erlenmeyer flask equipped with a rubber stopper (n = 3). 100 ml of water (Millipore quality, ELIX 3, Gradient A 10, pH 5.92 - 6.00) were added and 30 minutes of ultrasonic treatment was applied to the samples. Afterwards the samples were stirred in the dark for 24 hours at 37°C in a water bath. After the desired stirring time, each sample was filtered through a 0.2 µm membrane filter (Schleicher & Schuell, RC 0.2 µm Spartan 30/A) and concentrated on a Sep-Pak cartridge (Waters Sep-Pak cartridge C18, WAT051910). Then the cartridge was eluted with ethanol (gradient grade, Merck). The ciclesonide in the ethanol solution was then quantified by HPLC (LaChrom®-HPLC System Series 7000, column: Zorbax SB phenyl, eluent: water/ethanol 38 % : 62 % (v/v) isocratic, column temperature: 60°C, injection volume: 50 µL, UV-detection at 243 nm) against an external standard. Results are given in Figure 4.

### Example 5: Preparation of amorphous ciclesonide

For the preparation of amorphous ciclesonide about 0.5 g crystalline ciclesonide were dissolved in 70 ml 2-methyl-propan-2-ol (tert-butanol). The clear solution was quench cooled in liquid nitrogen under stirring (cooling rate at least 100 °C/min) and lyophilised (low pressure: 0.22 mbar) over 2 days, using a Freeze Dryer GAMMA 2-16 LSC from Martin Christ Gefriertrocknungsanlagen GmbH (37520 Osterode am Harz, Germany). The obtained amorphous ciclesonide is a light, voluminous powder. As tert-butanol was used as solvent for the lyophilisation process, it was of interest to quantify the amount of remaining tert-butanol in the lyophilised batches. Two quantification methods were established: 1) a thermogravimetrical method, which quantifies the tert-butanol content via a weight loss determination in the temperature range 76 - 134°C, and 2) a ¹H-NMR-spectrometrical method, which quantifies the tert-butanol content via the ratio of the integrals of the singulett proton signal of the three methyl-groups of tert-butanol (chemical shift: 1.27 ppm) and the signal of the olefinic H2 proton (chemical shift: 6.29 ppm) of ciclesonide in the ¹H NMR-spectra. The results of both methods were in good agreement and showed that less than 0.6 % (w/w) of tert-butanol was present in the samples used for the investigations.

## Claims

1. The compound of formula I 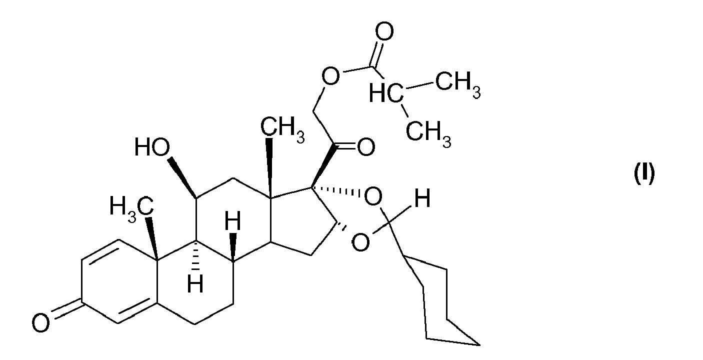 (ciclesonide) or a pharmaceutically acceptable solvate thereof being to more than 50 % (w/w) in amorphous form.

2. The compound according to claim 1 being to more than 95 % (w/w) in amorphous form.

3. The compound of claim 1 or 2, **characterized by** an X-ray diffraction pattern devoid of Bragg reflexes at 2Θ = 6.8° +/-0.3°, 14.4° +/-0.3°, 14.9° +/-0.3°, 16.8° +/-0.3°, 17.4° +/-0.3°, and 18.3° +/-0.3° which are present in the powder X-ray diffraction pattern of crystalline ciclesonide obtainable when using the Kα₁ line with a wavelength of 1.54056 A of a copper X-ray tube as radiation source.

4. The compound of any of the preceeding claims, **characterized by** a FT-Raman spectrum having a signal intensity ratio of I _{787 cm-1} / I _{803 cm-1} = 1.0 +/-0.2.

5. The compound of any of the preceeding claims, **characterized by** a DSC scan having an exothermal recrystallization signals below the melting point of crystalline ciclesonide.

6. The compound of any of the preceeding claims, **characterized by** a solubility in water at 37°C after 20 min of at least 150 µg/l.

7. The compound of any of claims 1 to 5, **characterized by** a solubility in water at 37°C after 20 min of at least 300 µg/l.

8. The compound of any of claims 1 to 5, **characterized by** a solubility in water at 37°C after 1 h of at least 150 µg/l.

9. The compound of any of claims 1 to 5, **characterized by** a solubility in water at 37°C after 1 h of at least 300 µg/l.

10. Process for preparing the compound according to any of claims 1-9, comprising the steps of:
a. providing a solution of the compound of formula I or a pharmaceutically acceptable solvate thereof,
b. quench cooling the solution of step (a), and
c. lyophilizing the quench cooled solution.

11. Process for preparing the compound according to any of claims 1-9, comprising the steps of:
a. providing a solution of the compound of formula I or a pharmaceutically acceptable solvate thereof, and
b. spray drying the solution of step (a).

12. Process according to claim 10 or 11, comprising the use of a solvent for preparing the solution of the compound of formula I or a pharmaceutically acceptable solvate thereof, the solvent being selected from tert-butanol and dioxan.

13. Process for preparing the compound according to any of claims 1-9, comprising the step of milling crystalline ciclesonide.

14. Amorphous form of the compound of formula I or a pharmaceutically acceptable solvate thereof, obtainable by a process according to any of claims 10-13.

15. Amorphous form of the compound of formula I or a pharmaceutically acceptable solvate thereof, obtained by a process according to any of claims 10-13.

16. Pharmaceutical composition, comprising a compound or a pharmaceutically acceptable solvate thereof according to any of claims 1-9 or claims 14-15 as active ingredient.

17. Compound or a pharmaceutically acceptable solvate thereof according to any of claims 1-9 or claims 14-15 for therapeutic use.

18. Compound or a pharmaceutically acceptable solvate thereof according to any of claims 1-9 or claims 14-15, or a pharmaceutical composition comprising a compound according to any of claims 1-9 or claims 14-15 or a pharmaceutically acceptable solvate thereof, for the treatment of respiratory diseases.

19. Use of a compound or a pharmaceutically acceptable solvate thereof according to any of claims 1-9 or claims 14-15 in the manufacture of a pharmaceutical composition for the treatment of respiratory diseases.
